# EUROPEAN PATENT SPECIFICATION

(11) **EP 0 236 477 B1**
(45) Date of publication and mention of the grant of the patent: **20.01.1993**
(21) Application number: 86906052.5
(22) Date of filing: 08.09.1986
(51) Int. Cl.: A61K 31/485

(54) **Use of 6-methylene-6-desoxy-N-cyclopropylmethyl-14-hydroxydihydronormorphine**
Verwendung von 6-Methylen-6-desoxy-N-cyclopropylmethyl-14-hydroxydihydronormorphin
Utilisation de méthylène-6 désoxy-6, N-cyclopropyl-méthyl-hydroxy-14-dihydronormorphine

(30) Priority: 06.09.1985 US 773213
(43) Date of publication of application: 16.09.1987
(73) Proprietor: BAKER NORTON PHARMACEUTICALS, INC., Miami Florida 33166 (US)
(72) Inventor: TUTTLE, Ronald, R. Key Pharmaceuticals, Inc., Miami, FL 33137 (US); HOWES, John Key Pharmaceuticals, Inc., Miami, FL 33137 (US)
(74) Representative: Werner, Hans-Karsten, Dr.Dipl.-Chem.
(86) International application number: US8601847
(87) International publication number: WO8701282

(56) References cited:
- EP-A- 0 140 367
- EP-A- 0 144 243
- FR-A- 2 160 957
- GB-A- 769 517
- US-A- 3 493 657
- US-A- 3 814 768
- US-A- 4 567 185
- J. of Pharmac. Sciences, vol. 73, no. 11, Nov. 1984, (US), R. Dixon et al., pp. 1645-1646
- Research Communications in Chemical Pathology and Pharmacology, vol. 13, no. 4, April 1976, (New York, US), R.D. Heilman et al., pp. 635-647
- Chemical Abstracts, vol. 103, no. 23, 9 Dec. 1985, (Columbus, Ohio, US), M.E. Michel et al., p. 61, abstract 189569b, Methods Find. Exp. Clin. Pharmacol. 1985, 7(4), 175-7
- J. of Medical Chemistry, vol. 18, no. 3, 1975 (US), E.F. Hahn et al., pp. 259-262
- Substance and Alcohol Actions/Misuse, vol. 5, no. 2, 1984, Pergamon Press Ltd. (US), M.J. Katovich et al., pp. 87-95
- Research Communications in Chemical Pathology and Pharmacology, vol. 42, no. 3, Dec. 1983, (US) J. Hsiao et al., pp. 449-454
- Chemical Abstracts, vol. 88, no. 1, 2 Jan. 1978, (Columbus, Ohio, US), E.S. Vizi et al., p. 167, abstract 167b, Congr. Hung. Pharmacol. Soc. (Proc) 1974 (Pub. 1976), 2(1, Symp. Analg.), 85-96
- Fed. Proceed., vol. 43, no. 4, 1984, C.B. Mash et al., p. 967, abstract 3987
- Goodman and Gilman's The Pharmalogical basis of therapeutics, 7th ed. 1985, Macmillan Publishing Co., (New York, US), pp. 524-527 and 573-574
- Anesthesiology, vol. 64, no. 2, Febr. 1986, T.J. Gal M.D. et al., pp. 175-180
- Clin. Pharmacol. Ther., vol. 39, no. 1, Jan. 1986, R. Dixon et al., pp. 49-53
- J. Clin. Pharmacol., vol. 26, no. 7, Sept./Oct. 1986, L.R.C. Moore et al., p. 558
- Clin. Pharmacol. ther., vol. 40, no. 5, Nov. 1986, T.J. Gal, M.D. et al., pp. 537-542

## Description

The invention is directed to use of the compound 6-methylene-6-desoxy-N-cyclopropylmethyl-14-hydroxydihydronormorphine to provide opioid antagonism while preventing renarcotization of the subject.

Opioid antagonists presently are used to counter the effects of opioid narcotics. The compound naloxone, a pure opioid antagonist, is used in treating opioid drug overdoses, for example. However, use of naloxone suffers from a disadvantage in that the active duration of naloxone is only about 45-90 minutes. Thus, renarcotization of the subject following administration of the naloxone can occur. This happens when the opioid is not metabolized as quickly as the naloxone. Thus, a subject apparently fully revived by treatment with injectable naloxone can later suffer from reappearing opioid effects, i.e., renarcotization, a condition which at best results in the nuisance of continued medical supervision and repeated injections of naloxone, and at worst is life-threatening if not recognized and treated.

The compound 6-methylene-6-desoxy-N-cyclopropylmethyl-14-hydroxydihydronormorphine is a known pure opioid antagonist. The compound is described in Fishman U.S. Patents 3,814,768 and 3,896,226.

Fishman '226 discloses a preferred oral dosage of 0.1-10.0 mg of 6-methylene-6-desoxy dihydro-morphine and -codeine derivatives per kg body weight, and mentions a narcotic antagonist effect persisting for 8-12 hours. A parenteral dose of 0.02-2 mg per kg body weight also is disclosed.

Hsiao and Dixon, Research Communications in Chemical Pathology and Pharmacology, Vol. 42, No. 3, pp.449-54, Dec. 1983, describe a process for detecting 6-methylene-6-desoxy-N-cyclopropylmethyl-14-hydroxydihydronormorphine in human plasma. The results show that a pharmacologically active concentration of 6-methylene-6-desoxy-N-cyclopropylmethyl-14-hydroxydihydronormorphine can remain in the plasma of a patient for three days.

R.D. Heimann et al. in "Research Communications in Chemical Pathology and Pharmacology", volume 13, no. 4, April 1976, pages 635 - 647 describe an evaluation of the hot plate technique to study narcotic antagonists. They found among others that the substance ORF 11676 is about four times more potent as an antagonist by the intravenous route than the structural nearest compound naltrexon. Both are significantly more potent than naloxone. There is, however, no indication that ORF 11676 also can be used as a long lasting and reliable drug antagonizing the narcotic effects of morphine, meperidine and fentanyl without the renarcotization being known to be dangerous and difficult to be handled. From naloxone this dangerous side effect was well-known and made the substance difficult to be handled.

This highly desirable properties are found especially if the substance is administered intravenously.

The present invention therefore provides the use of 6-methylen-6-desoxy-N-cyclopropylmethyl-14-hydroxydihydronormorphine for the manufacture of an intravenously injectable pharmaceutical composition antagonizing the narcotic effects of morphine, meperidine or fentanyl which were administered for the purpose of analgesia prior to the performance of a surgical or diagnostic procedure without renarcotization, said composition containing the active compound in an amount of 0.1 to 25.0 mg.

Preferably said composition contains the amount from 0.5 to 2.0 mg.

With this composition it is possible to treat a subject who has undergone opioid-induced general anesthesia, comprising injecting said subject with the compound 6-methylene-6-desoxy-N-cyclopropylmethyl-14-hydroxydihydronormorphine in an amount sufficient to antagonize the narcotic effects of the opioid anesthetic, the amount of 6-methylene-6-desoxy-N-cyclopropylmethyl-14-hydroxydihydronormorphine providing a sufficiently sustained narcotic antagonism so that renarcotization of the subject is prevented.

Furtheron, it is possible to treat a subject who is suffering from narcotic effects of an opioid drug overdose, comprising injecting said subject with the compound 6-methylene-6-desoxy-N-cyclopropylmethyl-14-hydroxydihydronormorphine in an amount sufficient to relieve the narcotic effects of the opioid drugs, the amount of 6-methylene-6-desoxy-N-cyclopropylmethyl-14-hydroxydihydronormorphine providing a sufficiently sustained narcotic antagonism so that renarcotization of the subject is prevented.

Furtheron, it is possible to treat a patient who has undergone opioid analgesia for a surgical or diagnostic procedure, comprising injecting said subject with the compound 6-methylene-6-desoxy-N-cyclopropylmethyl-14-hydroxydihydronormorphine in an amount sufficient to antagonize the narcotic effects of the opioid analgesic, the amount of 6-methylene-6-desoxy-N-cyclopropylmethyl-14-hydroxydihydronormorphine providing a sufficiently sustained antagonism so that renarcotization of the subject is prevented.

Furtheron, it is possible to treat a subject undergoing a surgical or diagnostic procedure, comprising administering to the subject an opioid analgesic in an amount sufficient to relieve discomfort from the surgical or diagnostic procedure; performing the surgical or diagnostic procedure; and injecting the subject with the compound 6-methylene-6-desoxy-N-cyclopropylmethyl-14-hydroxydihydronormorphine after completion of the procedure, the 6-methylene-6-desoxy-N-cyclopropylmethyl-14-hydroxydihydronormorphine being injected in an amount sufficient to antagonize the narcotic effects of the opioid analgesic and to provide sustained narcotic antagonism so that renarcotization of the subject is prevented and the subject may be released from a physician's attendance upon the injection of 6-methylene-6-desoxy-N-cyclopropylmethyl-14-hydroxydihydronormorphine taking effect.

Furtheron, it is possible to prevent respiratory depression in a subject undergoing epidural opioid regional analgesia, comprising injecting said subject with an amount of 6-methylene-6-desoxy-N-cyclopropylmethyl-14-hydroxydihydronormorphine sufficient to antagonize respiratory depressive effects of the epidural opioid analgesic.

Furtheron, it is possible to antagonize opioid narcotic effects in a baby whose mother is given an opioid analgesic during delivery, comprising administering the compound 6-methylene-6-desoxy-N-cyclopropylmethyl-14-hydroxydihydronormorphine to the baby by injection through the umbilical vein, the amount of 6-methylene-6-desoxy-N-cyclopropylmethyl-14-hydroxydihydronormorphine being sufficient to antagonize the narcotic effects and provide sustained narcotic antagonism so that renarcotization is prevented.

Furtheron, it is possible to treat a subject suffering from narcotic effects of endogenous opioids, comprising injecting the subject with the compound 6-methylene-6-desoxy-N-cyclopropylmethyl-14-hydroxydihydronormorphine in an amount sufficient to antagonize the narcotic effects of the endogenous opioid, the amount of 6-methylene-6-desoxy-N-cyclopropylmethyl-14-hydroxydihydronormorphine providing a sufficiently sustained narcotic antagonism so that renarcotization of the subject is prevented.

The composition can be used in form of a kit which comprises:
(a) An opioid analgesic suitable for providing relief of discomfort in a subject undergoing a surgical or diagnostic procedure.
(b) an intravenous dosage form containing a dosage unit capable upon administration to the subject of providing a continuous prolonged presence in the blood stream of a pure opioid antagonist for a period of at least about eight hours which comprises in a pharmaceutically inert diluent 6-methylene-6-desoxy-N-cyclopropylmethyl-14-hydroxydihydronormorphine in an amount sufficient to antagonize the narcotic effects of the opioid analgesic in the subject over said prolonged period, whereby a sufficiently sustained narcotic antagonism effect is provided over said prolonged period, whereby during said prolonged period renarcotization of the subject is avoided; and
(c) instructions on the administration of the active ingredient for said prolonged presence.

Finally it is possible to antagonize opioid narcotic effects in a subject having need of opioid narcotic antagonism, comprising injecting said subject with the compound 6-methylene-6-desoxy-N-cyclopropylmethyl-14-hydroxydihydronormorphine in an amount sufficient to antagonize the opioid narcotic effects in the subject, the amount of the compound 6-methylene-6-desoxy-N-cyclopropylmethyl-14-hydroxydihydronormorphine being sufficient to provide a sustained narcotic antagonism such that renarcotization of the subject is prevented for a prolonged period of at least about eight hours.

The drawing is a graph showing results obtained in the tests described below.

The compound 6-methylene-6-desoxy-N-cyclopropylmethyl-14-hydroxydihydronormorphine is a specific antidote for opioid narcosis. Like the compound naloxone, 6-methylene-6-desoxy-N-cyclopropylmethyl-14-hydroxydihydronormorphine is a pure opioid antagonist, exerting no opioid effects. Like naloxone, it is effective against both endogenous opioids, e.g. endorphins, and natural or synthetic exogenous opioids, e.g. morphine and Demerol.

Intravenous injection of a subject with 6-methylene-6-desoxy-N-cyclopropyl-methyl-14-hydroxydihydronormorphine provides fast acting and sustained opioid antagonism. These properties will make 6-methylene-6-desoxy-N-cyclopropylmethyl-14-hydroxydihydronomorphine desirable for uses where naloxone presently cannot be used, as well as improve treatment in fields where naloxone presently is used such as the treatment of drug overdose cases. The long duration of the opioid antagonism also decreases the need for physician attendance and medical supervision.

The amount of 6-methylene-6-desoxy-N-cyclopropylmethyl-14-hydroxydihydronormorphine administered to a subject will be from about 0.1-25 mg. It is preferred to give the 6-methylene-6-desoxy-N-cyclopropylmethyl-14-hydroxydihydronormorphine in doses of about 1 mg with repeated injections if necessary. The injectable compositions are made by dissolving the active ingredient in a suitable carrier, such as water or saline. Further components such as preservatives and acid for pH adjustment can be added if desired. The order of addition to the carrier is not important.

One specific intravenously injectable composition contemplated includes the following per each ml of injectable composition: 1.108 mg 6-methylene-6-desoxy-N-cyclopropylmethyl-14-hydroxydihydronormorphine hydrochloride; 1.8 and 0.2 mg respectively of the preservatives methylparaben and propylparaben; 9 mg USP grade NaCl; HCl to provide a pH of 3.9; and sterile water. It should be understood that the term 6-methylene-6-desoxy-N-cyclopropylmethyl-14-hydroxydihydronormorphine is used in this application to refer to the compound itself as well as pharmaceutically effective derivatives such as the acid addition salc noted in the listing above.

It is desirable for the composition to be stored in containers ready for use, such as ampules or prefilled syringes containing about 1 ml of the composition outlined above. Such containers can be made part of a kit which would include the container as well as instructions for treatment. The kit also could hold containers of an opioid analgesic to be used in minor surgical or diagnostic proceedings (described below) if desired.

There are a number of remedial uses for intravenously injectable 6-methylene-6-desoxy-N-cyclopropylmethyl-14-hydroxydihydronormorphine, i.e. for reversing the effects of previously administered opioids. Intravenously injectable 6-methylene-6-desoxy-N-cyclopropylmethyl-14-hydroxydihydronormorphine could be used for treatment of victims of opioid drug overdose. Currently, naloxone is injected to revive such overdose victims. However, the short duration of naloxone's opioid antagonistic effect can result in renarcotization of the patient, sometimes leading to loss of life. The present compound's increased duration of antagonistic activity (at least 6, preferably 8-9 hours) helps prevent renarcotization until the opioid has been metabolized. The intravenously injectable composition could be distributed in the form of pre-filled syringes with suitable instructions. The safety of the present compound would allow the inclusion of a sufficient amount of active ingredient so that self-administration could be possible.

Intravenously injectable 6-methylene-6-desoxy-N-cyclopropylmethyl-14-hydroxydihydronormorphine expands the use of opioids for general anesthesia. At present, opioid general anesthesia is reserved for high-risk major surgery such as open heart surgery. One main reason why opioid general anesthesia is not used for other types of major surgery is the problem of dealing with potential post-operative respiratory depression from the opioid. Intravenously injection of 6-methylene-6-desoxy-N-cyclopropylmethyl-14-hydroxydihydronormorphine to revive the patient helps to alleviate this problem by providing opioid antagonism (i.e., against respiratory depression) for a period of time sufficient for the opioid anesthetic to be metabolized.

Intravenously injectable 6-methylene-6-desoxy-N-cyclopropylmethyl-14-hydroxydihydronormorphine is also useful in diagnostic and minor surgical procedures which are painful or anxiety producing, and thus require some analgesic during the procedure, but require no analgesia when the procedure is finished. Such procedures include, for example, lancing boils, setting dislocated shoulders, various kinds of dental work, certain radiological procedures, endoscopies of the gastrointestinal tract, endoscopies of the urinary system and bronchoscopies. Intravenously injectable 6-methylene-6-desoxy-N-cyclopropylmethyl-14-hydroxydihydronormorphine will allow the use of opioid analgesics for such procedures, followed by injection of the 6-methylene-6-desoxy-N-cyclopropylmethyl-14-hydroxydihydronormorphine to bring the patient out of the analgesia. 6-methylene-6-desoxy-N-cycloporpylmethyl-14-hydroxydihydronomorphine reduces the possibility of renarcotization so that the patient can go home without having to wait for the analgesic to wear off. This makes such surgical procedures much more convenient and less costly for patients. Further, the procedures can be conducted with sufficient analgesia to provide optimum patient comfort.

Intravenous injection of 6-methylene-6-desoxy-N-cyclopropylmethyl-14-hydroxydihydronormorphine also can be used for treating newly delivered babies. Many hospitals administer Demerol to delivering mothers. The Demerol is transmitted to the baby, making it dopey. Injection of 6-methylene-6-desoxy-N-cyclopropyl-methyl-14-hydroxydihydronormorphine through the umbilical vein upon delivery helps counter the opioid narcotic effects of the Demerol in the infant.

Intravenously injectable 6-methylene-6-desoxy-N-cyclopropylmethyl-14-hydroxydihydronormorphine also is useful in remedying the effects of endogenous opioids. Thus, it is useful in treating shock and neural trauma.

Intravenously injectable 6-methylene-6-desoxy-N-cyclopropylmethyl-14-hydroxydihydronormorphine also finds use in prophylactic applications, for example during surgery involving epidural opioid regional analgesia. Epidural opioid regional analgesia involves application of an opioid directly to the spinal cord in a high concentration. This procedure produces complete relief of pain supplied by pain conducting nerves below the site of epidural application. The result is like that of a spinal done with local anesthetics, except the disadvantage of paralysis is not present. A major problem with the epidural opioid technique is unpredictable respiratory depression which can occur if the opioid migrates from the spinal cord to the brain. Intravenous injection of 6-methylene-6-desoxy-N-cyclopropylmethyl-14-hydroxydihydronormorphine would provide protection against this problem through the long-lasting opioid antagonistic properties, which are sufficient to counteract any opioid migrating to the brain. However, the 6-methylene-6-desoxy-N-cyclopropylmethyl-14-hydroxydihydronormorphine injected would not significantly affect the high concentration of the opioid provided at the spinal cord.

The unexpected long duration of action of nalmefene makes it of value in the treatment of pets, zoo animals and commercially important animals such as cattle and sheep.

Because of nalmefene's long duration of action it is possible to give animals very large doses of opioids that will allow painful procedures to be done on these animals. After completion of the procedure the opioid induced narcosis can be rapidly and completely reversed. In contrast to other opioid antagonists available i.e. naloxone, with nalmefene there is no fear of renarcotization.

Particular applications are:

### Pets

The veterinary treatment of injured dogs. For example, a dog hit by a car that is in great pain can have pain relieved by large doses of an opioid and any surgical repairs can be done while the dog is under the influence of the opioid. Then the opioid can be reversed by nalmefene and the owner can take home a fully revived pet.

### Zoo Animals

These large animals such as deer, springbuck, onyx and rhinoceros are immobilized by the zoo's veterinary staff with opioids delivered from dartguns. This immobilization permits the vet to carry out minor surgical procedures. Nalmefene, as in the dog, will rapidly and completely reverse the opioid without fear of renarcotization. Nalmefene is lifesaving in these animals because if they renarcotize they can become hyperthermic and die.

### Cattle and Sheep

Branding of these animals is painful and inhumane. Branding could be carried out humanely by doing it while the animal is heavily narcotized with an opioid. As in the above two cases, the narcotic can be reversed with nalmefene in cows or sheep rapidly and completely without fear of renarcotization. Thus the animals can be immediately returned to the herd.

### Example

The study was designed to test the duration of 6-methylene-6-desoxy-N-cyclopropylmethyl-14-hydroxydihydronormorphine action by pretreating subjects with the antagonist and then challenging with periodic doses of a short-acting opioid agonist (fentanyl).

Methods. Six healthy males (ages 23-28) were pretreated in random double-blind fashion on each of four separate days with a saline placebo, 0.5 mg, 1.0 mg, or 2.0 mg 6-methylene-6-desoxy-N-cyclopropylmethyl-14-hydroxydihydronormorphine intravenously. Subjects were tested before and after this pre-treatment, and following opioid challenge with each of five doses of fentanyl (2 µg/kg) at 1, 2, 4, 6, and 8 hours afterwards. Respiratory depression was identified by the CO₂ rebreathing method of Read. Ventilatory and occlusion pressure responses were analyzed by relating slopes of the increased minute ventilation (VE) and occlusion pressure (P_{0.1}) to end -tidal CO₂, and by recording VE and P_{0.1} at a fixed level of increased CO₂ (60 mmHg) during rebreathing. Analgesia to experimental pain was assessed by recording the time to onset of unbearable pain (tolerance) during submaximal tourniquet-induced ischemia.

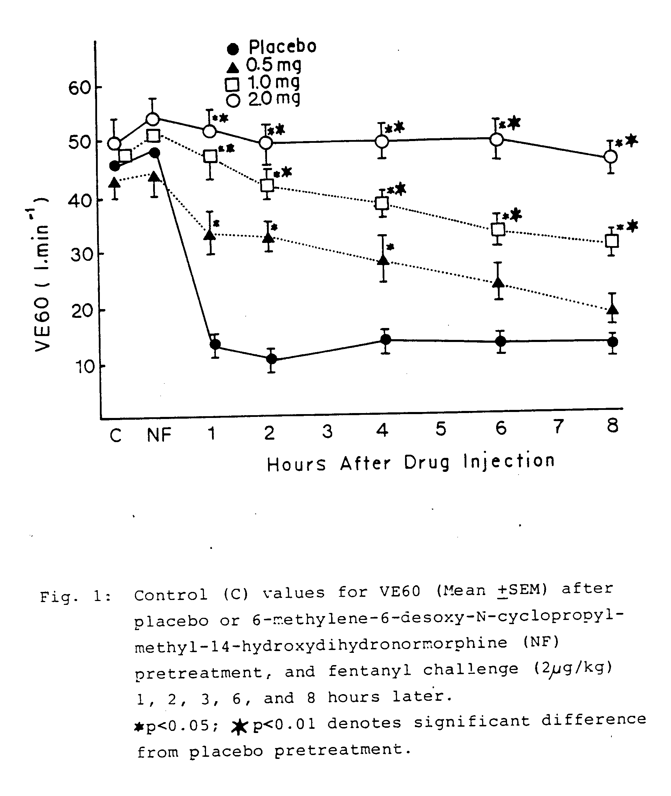

Results. At one hour following placebo pretreatment, fentanyl produced nasal itching, mild nausea, drowsiness, and marked respiratory depression compared to the control state (Table 1) below. Both VE60 (29% of control) and P_{0.1}60 (41% of control) were significantly decreased (P<_{0.01}) as were the sloped of the ventilatory and occlusion pressure responses (VE/PCO₂, P_{0.1}/PCO₂) which were 51 and 55% of control, respectively. Each subsequent fentanyl dose produced a similar degree of respiratory depression as illustrated by VE60 (Fig.1). Pretreatment with 2.0 mg 6-methylene-6-desoxy-N-cyclopropylmethyl-14-hydroxydihydronormorphine completely prevented the subjective and respiratory effects of fentanyl for the entire 8 hours of the experiment. 6-methylene-6-desoxy-N-cycloporpylmethyl-14-hydroxydihydronormorphine (1.0 mg) significantly blunted the respiratory depression over the same period when compared to placebo pretreatment, but VE60 values at 6 and 8 hours were depressed significantly (P< 05) to 66 and 61% of control. The antagonist effects of the lowest 6-methylene-6-desoxy-N-cyclopropylmethyl-14-hydroxydihydronormorphine dose (0.5 mg) persisted for about 4 hours, at which time VE60 was 64% of control.

In the absence of 6-methylene-6-desoxy-N-cyclopropylmethyl-14-hydroxydihydronormorphine, each fentanyl dose produced consistent increases in tolerance to pain (44-55% above control). 6-methylene-6-desoxy-N-cyclopropylmethyl-14-hydroxydihydronormorphine pretreatment abolished this analgesic response in a dose-related time course which mirrored the respiratory effects almost exactly.

**Table 1**

| Ventilatory Responses | | |
|---|---|---|
| | Control | Fentanyl |
| VE60 (1.min⁻¹) | 45.9 (6.3) | 13.4* (2.3) |
| P_{0.1}60 (cm H₂O) | 8.0 (1.2) | 3.3* (0.4) |
| VE/PCO₂ (1.min⁻¹.mmHg⁻¹) | 3.36 (0.47) | 1.73* (0.26). |
| P_{0.1}/PCO₂ (cm H₂0.mmHg⁻¹) | 0.58 (0.09) | 0.32* (0.07) |
| Values are Mean ± SEM for six subjects | | |

| | | |
|---|---|---|
| *p<0.01 denotes significant difference from control. | | |

## Claims

1. Use of 6-methylen-6-desoxy-N-cyclopropylmethyl-14-hydroxydihydronormorphine for the manufacture of an intravenously injectable pharmaceutical composition antagonizing the narcotic effects of morphine, meperidine or fentanyl which were administered for the purpose of analgesia prior to the performance of a surgical or diagnostic procedure without renarcotization, said composition containing the active compound in an amount of 0.1 to 25.0 mg.

2. Use according to claim 1 characterized in that the amount is from 0.5 to 2.0 mg.

## Patentansprüche

1. Verwendung von 6-Methylen-6-desoxy-N-cyclopropylmethyl-14-hydroxydihydronormorphin für die Herstellung einer intravenös injizierbaren pharmazeutischen Zusammensetzung, die die narkotischen Wirkungen von Morphin, Meperidin oder Fentanyl antagonisiert, die zum Zweck der Analgesie vor der Durchführung eines chirurgischen oder diagnostischen Vorgehens ohne Renarkotisierung verabreicht wurden, wobei die Zusammensetzung den Wirkstoff in einer Menge von 0,1 bis 25,0 mg enthält.

2. Verwendung nach Anspruch 1, dadurch gekennzeichnet, daß die Menge von 0,5 bis 2,0 mg reicht.

## Revendications

1. Utilisation de méthylène-6 désoxy-6, N-cyclopropylméthyl-hydroxy-14-dihydronormorphine pour la fabrication d'un composé pharmaceutique pour l'injection intraveineuse, qui antagonise les effets narcotiques du morphine, méperidine ou fentanyl, qui étaient administrés à propos d'analgésie avant l'exécution d'un procédé chirurgical ou diagnostique sans rénarcotisation, le composé contenant la substance active dans une quantitée de 0,1 à 25,0 mg.

2. Utilisation suivant la revendication 1, caractérisée en ce que la quantitée est de 0,5 à 2,0 mg.
